# EUROPÄISCHE PATENTANMELDUNG

(11) **EP 4 528 269 A1**
(43) Veröffentlichungstag der Anmeldung: **26.03.2025**
(21) Anmeldenummer: 23199337.9
(22) Anmeldetag: 25.09.2023
(51) Int. Cl.: G01N 33/04

(54) **VERFAHREN ZUR BEURTEILUNG DER STABILITÄT UND QUALITÄT VON MILCHPRODUKTEN SOWIE PFLANZLICHEN MILCHALTERNATIVEN ÜBER DIE LAGERZEIT**

(71) Anmelder: DMK Deutsches Milchkontor GmbH, 27404 Zeven (DE)
(72) Erfinder: BODE, Katja, 21279 Hollenstedt (DE); SCHULZ, Maren Christina, 27412 Wilstedt (DE); ZINK, Ralf, 26160 Bad Zwischenahn (DE)
(74) Vertreter: Fabry, Bernd

(57) **Zusammenfassung**

Vorgeschlagen wird ein Verfahren zur Beurteilung der Stabilität und Qualität von Milchprodukten, Produkten auf primärer und hauptsächlicher Basis von Milch inklusive von Käsen und pflanzlicher Milchalternativen über die Lagerzeit, bei dem man Milchprodukte Produkte auf primärer und hauptsächlicher Basis von Milch inklusive von Käsen oder pflanzlichen Milchalternativen unter definierten Bedingungen altert, regelmäßig Proben entnimmt und diese an Hand verschiedener Parameter analysiert. Die Datensätze werden mit Hilfe Principal Component Analysis (PCA) und der Partial Least Square (PLS) Kalibrierungsmethode untersucht. Die dabei berechneten Altersangaben werden mit dem tatsächlichen Probenalter verglichen und liefern eine Kalibrierungsgerade, mit deren Hilfe das Alter unbekannter Proben ermittelt werden kann.

## Beschreibung

### GEBIET DER ERFINDUNG

Die vorliegende Erfindung befindet sich auf dem Gebiet der Lebensmitteltechnologie und betrifft ein Verfahren zur frühzeitigen Beurteilung der Stabilität und Qualität, insbesondere des Alters von Milchprodukten, Produkten auf primärer und hauptsächlicher Basis von Milch inklusive von Käsen oder deren pflanzlichen Milchalternativen unter Erhebung definierter Qualitäts- und Zusammensetzungsdaten und deren statistischer Auswertung.

### TECHNOLOGISCHER HINTERGRUND

Eine der größten Anforderungen in der Lebensmittelindustrie besteht darin, ihre Produkte nicht nur qualitativ einwandfrei herzustellen und in Verkehr zu bringen, sondern diese Qualität und Technofunktionalität auch für einen ausreichend langen Zeitraum sicherzustellen. Neben der mikrobiologischen Haltbarkeit, die durch geeignete Verfahren, wie Erwärmen, aseptisches Abfüllen und Verpackung, sowie Konservieren gewährleistet werden kann, müssen insbesondere auch chemische oder physikalische Veränderungen des Füllgutes während der Lagerzeit vermieden werden, da diese im günstigsten Fall nur zu Aromaveränderungen führen können, im ungünstigsten Fall jedoch die Nahrungsmittel verderben und technofunktional negativ beeinflussen. Einer der wichtigsten Faktoren, die die Qualität von Lebensmitteln beeinflussen, stellt die Permeation von Stoffen, wie insbesondere Sauerstoff, durch das Packmittel dar, die infolge von Oxidation Farbe, Geschmack des Lebensmittels, Technofunktionalität oder alle drei Faktoren beeinträchtigen kann. Die verwendeten Verpackungen müssen daher einen optimalen Produkt- und Funktionalitätsschutz gewähren und sowohl im Hinblick auf die Materialauswahl als auch das Verpackungsdesign hohen packgutspezifischen Anforderungen genügen. Insbesondere müssen innerhalb der deklarierten Mindesthaltbarkeit eine einwandfreie Qualität und Technofunktionalität des Produkts sichergestellt sein.

Zur Ermittlung der Qualität, Funktionalität und daraus abgeleitet der Mindesthaltbarkeit stehen neben Materialprüfungstests und theoretischen Berechnungsmodellen auch Standardqualifikationstests, wie etwa Produktlagertests in Echtzeit zur Verfügung. Dabei werden die zu untersuchenden Packmittel abgefüllt und über den Zeitraum der dafür vorgesehenen Mindesthaltbarkeit unter kontrollierten Bedingungen gelagert. Dies bedeutet allerdings, dass eine hinreichend gesicherte Aussage über die Tauglichkeit eines Verpackungsdesigns für ein bestehendes Produkt oder im Umkehrschluss einer Produktneuentwicklung für eine bestehende Verpackungsart erst nach Ablauf der vorgesehenen Mindesthaltbarkeit getroffen werden kann. In dieser sehr langen Testzeit von z.T. mehreren Monaten zeigt sich der wesentliche Nachteil dieser Testmethode direkt auf. Bedingt durch die immer größer werdende Produktvielfalt, zunehmende Transportwege und immer kürzer werdenden Produktzyklen bei gleichzeitig hoher Erwartung des Verbrauchers bezüglich Produktqualität und -sicherheit besteht im Wettbewerb der Anbieter von Produkten die Notwendigkeit zur Reduzierung der benötigten Entwicklungszeit.

### STAND DER TECHNIK

Aus dem Stand der Technik sind bereits einschlägige Verfahren bekannt, mit deren Hilfe man die Haltbarkeit von Lebensmitteln in einer Verpackung indirekt und ungenau bestimmen kann. So wird beispielsweise in der EP 1626275 B1 (WILD) ein Verfahren zur Bewertung der Produkthaltbarkeit in einem Packmittel vorgeschlagen, das die folgenden Schritte umfasst:
a) Einbringen eines mit Füllgut gefüllten, verschlossenen Packmittels (4) in eine Überdrucckammer (1),
b) Lagern des Packmittels (4) in der Überdruckkammer (1) über einen Zeitraum t₁ bei Überdruck p₁ und einer bestimmten Temperatur T₁ in einer Testgasatmosphäre, wodurch eine definierte Menge Q an Testgas in das Packmittel permeiert,
c) Lagern des Packmittels (4) über einen bestimmten Zeitraum tz unter Wärme und/oder Lichteinfluss, und
d) analytische und/oder sensorische Untersuchung des Füllguts.

Nachteilig bei dem Verfahren ist der hohe Apparateaufwand, zudem lässt das Funktionsprinzip - Nachbildung der in das Füllgut eingebrachten Sauerstoffmenge und Beschleunigung der Reaktion durch erhöhten Druck- letztlich nur eine sehr grobe Schätzung zu, die für eine qualitative Aussage vielfach ungeeignet ist, zumal bekannt ist, dass für viele Polymer/Gas-Systeme Löslichkeits- und Diffusionskoeffizient der permeirenden Substanz im Polymer (z.B. einer Plastikflasche) von Konzentration und Druck abhängig werden, wenn die Untersuchung unterhalb der Glastemperatur Tg der Polymere und/oder der kritischen Temperatur des Gases durchgeführt werden **[**MÜLLER, K. "Sauerstoff-Durchlässigkeit von Kunststoffflaschen und Verschlüssen" Dissertation TU München (2003**)]**

Eine völlig andere Methode, die Haltbarkeit eines verpackten Lebensmittels für den Verbraucher optisch anzuzeigen, ist Gegenstand der Schutzrechte EP 2378354 B1**,** EP 2581785 B1 sowie WO 2009 056591 A1 (UNI MÜNSTER). Diese betreffen eine Sensorvorrichtung, konkret einen elektrochemischen Prozessor, der mit dem Öffnen der Verpackung kurzgeschlossen wird. Es bildet sich eine Aluminiumoxidschicht, deren Fortschreiten proportional zur Haltbarkeit des Produktes verläuft. Eine Temperaturerhöhung führt beispielsweise dazu, dass die Oxidbildung beschleunigt wird und spiegelt damit natürlich auch den abnehmenden Frischegrad des Produktes wider. Diese Lösung macht es indes erforderlich, jede einzelne Verpackung mit einem separaten Sensor auszustatten, was ebenfalls technisch aufwendig ist und häufig instabile Ergebnisse bei Mehr- oder Einfachöffnungen aufzeigen kann.

EP 3875953 B1 (DMK) beschreibt einen beschleunigten Lagertest zur Bestimmung von Qualität und Haltbarkeit von Lebensmitteln, bei dem eine erste Probe bei einer Temperatur T1 und eine gleichartige zweite Probe bei einer deutlich höheren Temperatur T2 gelagert werden, wobei man die Proben sensorisch beurteilt und über eine Kalibrierkurve miteinander verglichen werden. Da diese sensorische Beurteilung von Produkten stets eine subjektive Komponente enthält, wäre eine Methode, die ausschließlich auf objektiven Parametern beruht vorteilhafter.

### AUFGABE DER ERFINDUNG

Daher hat die Aufgabe der vorliegenden Erfindung darin bestanden, ein Testverfahren bereitzustellen, welches durch Rückgriff auf ein Kalbrierungsdiagramm erlaubt, bestimmte Eigenschaften, wie etwa das Alter oder die Qualität eines unbekannten Milchproduktes, Produkten auf primärer und hauptsächlicher Basis von Milch inklusive von Käsen oder deren Milchalternativprodukten schnell und verlässlich anzugeben.

### BESCHREIBUNG DER ERFINDUNG

Die Erfindung betrifft ein Verfahren zur Beurteilung der Stabilität und Qualität von Milchprodukten, Produkten auf primärer oder hauptsächlicher Basis von Milch inklusive von Käse sowie pflanzlicher Milchalternativen über die Lagerzeit, umfassend oder bestehend aus den folgenden Schritten
(a) Bereitstellen eines ersten Satzes von Proben (P1.0, P2.0 ... Pn.0) eines Milchproduktes, eines Produktes auf primärer oder hauptsächlicher Basis von Milch inklusive von Käse, oder einer pflanzlichen Milchalternative;
(b) Alterung der Proben aus Schritt (a) über definierte Zeiträume unter Erhalt eines n-ten Probensatzes (P1.1, P1.2 ... P2.1, P2.2 ... Pn.x);
(c) Erstellen eines Sets von Prüfparametern;
(d) Anwendung der vorgegebenen Prüfparameter auf jede der einzelnen Proben des ersten und n-ten Probensatzes unter Erzeugung eines Datensatzes;
(e) Überprüfung des Zusammenhangs der Daten im Datensatz mit Hilfe der Principal Component Analysis (PCA) unter Erhalt eines Koordinatensystems (Score-Plot) und Ermittlung der größten Varianz der Daten als erster Komponente und der zweitgrößten Varianz der Daten als zweiter Komponente;
(f) Durchführung einer Partial Least Square Analyse (PLS) mit den Daten.
(g) Generierung eines Partial Least Square (PLS) Kalibrierungsmodells unter Erhalt eines PLS-Antwortdiagramms (Kalibriergerade), wobei die so berechnete Antwort für jeden Datenpunkt gegen die zugehörige gemessene Antwort jedes Datenpunktes in Relation gesetzt wird; sowie
(h) Beurteilung einer fremden Probe bezüglich des festgelegten Beurteilungskriteriums an Hand der Kalibrierungsgraden.

Überraschenderweise wurde gefunden, dass insbesondere das Alter und damit die Qualität eines Milchproduktes, Produkte auf primärer und hauptsächlicher Basis von Milch inklusive von Käsen oder pflanzlicher Milchalternative leicht und zuverlässig auf Basis von ausgewählten chemischen und physikalischen Daten ermittelt werden kann, die im Rahmen der Qualitätskontrolle ohnehin üblicherweise erhoben werden.

Das erfindungsgemäße Verfahren gliedert sich in vier Abschnitte:
- Auswahl geeigneter Analyseparameter
- Datengenerierung
- Überprüfung des Zusammenhangs der Daten durch PCA sowie
- Berechnung des Produktalters mittels PLS.

### Milchprodukte, Produkte auf primärer und hauptsächlicher Basis von Milch inklusive von Käsen oder deren pflanzlicher Milchalternativen

Die Auswahl der Milchprodukte, Produkte auf primärer und hauptsächlicher Basis von Milch inklusive von Käsen oder pflanzlicher Milchalternativen, auf die das erfindungsgemäße Verfahren angewendet werden kann, ist an sich unkritisch. Typischerweise sind die Produkte ausgewählt aus der Gruppe, die gebildet wird von Vollmilch, Magermilch, UHT-Milch, Milchpulvern, Sahne, Molke, Quark, Käse und Joghurt, aber auch Produkte auf primärer und hauptsächlicher Basis von Milch, wie beispielsweise zahlreiche Süßspeisen. Ebenfalls umfasst sind deren pflanzlichen Milchalternativen wie u.a. pflanzliche Drinks, Spreads, Sahnealternativen, Joghurtalternativen und Käsealternativen.

### Probensätze

Bevor der Datensatz erstellt werden kann, müssen zunächst die Proben vorbereitet werden. Der erste Probensatz umfasst die Proben zum Zeitpunkt t=0, es handelt sich also um die frischen Proben, die im Folgenden als P1, P2, P3 bis Pn bezeichnet werden. Der erste Datensatz umfasst dabei üblicherweise 3 bis 10 und vorzugsweise 4 bis 7 Proben, wobei diese in Gebinde abgepackt sind die typisch ein Volumen von 10 ml bis 100 L aufweisen.

Um den zweiten und jeden weiteren ("n-ten") Probensatz herzustellen, werden die ursprünglichen Proben unter definierten Bedingungen gelagert ("gealtert"), vorzugsweise abgedunkelt bei Temperaturen von 4 bis 20 °C. Die Lagerzeit kann einen Zeitraum von 1 bis 25 Wochen umfassen, sinnvollerweise werden eher längere Lagerzeiten von 4 bis 22 Wochen realisiert. Während dieser Zeit werden in regelmäßigen zeitlichen Abständen, typischerweise alle 1-2 Wochen, weitere Proben entnommen. Nach dem ersten Alterungszeitraum von 2 Wochen wird die aus der ursprünglichen Probe 2 die gealterte Probe 2 nach dem 1. Alterungszeitraum mit der Bezeichnung P2.1. Eine Probe P4.11 wäre dann die Bezeichnung für die ursprüngliche Probe 4, die 11 Alterungszeiträume durchlaufen hat, also 22 Wochen alt ist.

### Prüfparameter

Ein wesentliches Merkmal des erfindungsgemäßen Verfahrens ist die Auswahl der Prüfparameter auf deren Grundlage die Bewertung der Proben erfolgen soll. In Betracht kommen dafür vorzugsweise die Folgenden:
- gelöste Sauerstoffmenge
- pH-Wert
- Instabilitätsindex (Lumisizer)
- Helligkeitsfarbwert weiß/schwarz (L*)
- Farbkoordinate rot/grün (a*)
- Farbkoordinate gelb/blau (b*)
- freier Aminostickstoff (PAN)
- Ammoniakgehalt
- Harnstoffgehalt
- Calciumgehalt
- Säuregehalt.
- rheologisches Verhalten
- Festigkeit
- Syneräsegrad
- thermisches Verhalten (DSC)

Diese Aufzählung erhebt nicht den Anspruch auf Vollständigkeit. Je nach eingesetztem Milchprodukt, Produkte auf primärer und hauptsächlicher Basis von Milch inklusive von Käsen oder Milchalternativen ist es grundsätzlich möglich, weitere Parameter zu definieren. Die Aussagekraft des erfindungsgemäßen Verfahrens ist von der Auswahl und Anzahl der Parameter, auf die die Produkte untersucht werden, abhängig. Es hat sich als sachdienlich erwiesen, mindestens 5, vorzugsweise mindestens 7 Parameter auszuwählen. Je größer der Umfang des Sets ist, umso weniger wichtig ist die Aussagefähigkeit eines einzelnen Parameters.

Die meisten Parameter, wie pH-Wert, die gelöste Menge an Sauerstoff sowie der Gehalt an freiem Aminostickstoff, Harnstoff, Calcium und Säuren lässt sich mit den üblichen analytischen Verfahren ermitteln, die dem Fachmann aus der Lebensmittelanalytik und Milchwissenschaft notorisch bekannt sind und die hier nicht näher vorgestellt werden müssen.

Der Instabilitätsindex wird mit Hilfe des LUMiSizers bestimmt. Hierbei handelt es sich um ein Gerät, mit dem die Partikel- und Tröpfchengeschwindigkeit bei Aufrahmungs- und Sedimentationsprozessen misst und die Partikelgrößenverteilung gemäß DIN ISO 13318-2 bestimmt. Eine Beschreibung des Gerätes und der Technologie findet sich hier:
https://www.lum-gmbh.com/lumisizer de.html#:~:text=Der%20LUMiSizer%20ist%20das%20einzige,Systeme%20%E2%80%93%20Sie%20haben%20die%20Wahl

Der Helligkeitsfarbwert L* sowie die beiden Farbkoordinaten a* und b* gehören zum sogenannt L*a*b* Farbsystem, das auch als CIELAB-System bezeichnet wird. Es handelt sich um das heute gebräuchlichste System für die Farbmessung und hat in fast allen Anwendungsbereichen eine große Verbreitung gefunden. Es wurde 1976 als einer der gleichabständigen Farbräume von der CIE definiert, um dem Hauptproblem des ursprünglichen Yxy-Systems zu begegnen: Gleiche geometrische Differenzstrecken im x,y-Farbdreieck führen nicht zu empfindungsgemäß gleichen Farbunterschieden. Der Farbraum des L*a*b*-Systems ist durch die Helligkeit L* und die Farbkoordinaten a* und b* gekennzeichnet. Die Vorzeichen lassen die Farbrichtung erkennen: +a* deutet auf einen Rotanteil hin, -a* zeigt in Richtung Grün. Dementsprechend steht +b* für Gelb, und -b* für Blau. Im Koordinatenursprung (Achsenschnittpunkt) befindet sich ein neutrales Grau ohne jede Buntheit. Mit wachsenden a*b*-Werten, je weiter also der Farbort von der Mitte entfernt liegt, wird die Buntheit größer. Für die Ermittlung der Farbwerte hat sich im Rahmen des erfindungsgemäßen Verfahrens das Gerät CR400 bzw. 410 bewährt. Als weiterführende Informationen finden sich in der Verbraucherinformation "Exakte Farbkommunikation" der Firma Konica Minolta.

### Principal Component Analysis (PCA)

Der so erhaltene Datenpool wird im nächsten Schritt mit Hilfe der sogenannten "Principal Component Analysis" (PCA), die auch als Hauptkomponenten-Analyse bezeichnet wird, ausgewertet.

Dabei handelt es sich um ein etabliertes statistisches Verfahren zur Analyse großer Datensätze mit einer hohen Anzahl von Parametern pro Beobachtung, zur Verbesserung der Interpretierbarkeit von Daten unter Beibehaltung eines Höchstmaßes an Informationen und zur Visualisierung mehrdimensionaler Daten. Formal gesehen ist die PCA eine statistische Technik zur Reduzierung der Dimensionalität eines Datensatzes. Dies geschieht durch eine lineare Transformation der Daten in ein neues Koordinatensystem, in dem (der größte Teil) der Variation in den Daten mit weniger Dimensionen beschrieben werden kann als in den Ausgangsdaten. Im Rahmen des erfindungsgemäßen Verfahrens werden nur die ersten beiden Hauptkomponenten verwendet, um die Daten in zwei Dimensionen darzustellen und visuell Cluster von eng zusammenhängenden Datenpunkten zu identifizieren.

Bei der PCA-Datenanalyse wird die erste Hauptkomponente eines Satzes von p Variablen, von denen angenommen wird, dass sie gemeinsam normal verteilt sind, die abgeleitete Variable, die als lineare Kombination der ursprünglichen Variablen gebildet wird und die größte Varianz erklärt. Die zweite Hauptkomponente erklärt die größte Varianz in dem, was übrig bleibt, wenn der Effekt der ersten Komponente entfernt wird, und wir können fortfahren mit p Iterationen, bis die gesamte Varianz erklärt ist. Die PCA wird am häufigsten verwendet, wenn viele der Variablen stark miteinander korreliert sind und es wünschenswert ist, ihre Anzahl auf einen unabhängigen Satz zu reduzieren. Die erste bzw. zweite Hauptkomponente kann entsprechend als eine Richtung definiert werden, die die Varianz der projizierten Daten maximiert. Die PCA ist die einfachste der echten eigenvektorbasierten multivariaten Analysen und ist eng mit der Faktorenanalyse verwandt. Eine ausführliche Darstellung der Methode findet sich beispielsweise in https://en.wikipedia.org/wiki/Principal component analysis

Kurz zusammengefasst liefert die PCA ein Koordinatensystem, bei dem für jeden Datenpunkt die Komponente mit der größten Varianz oder Abweichung ("Erste Komponente") gegen die Komponente mit der zweitgrößten Varianz ("Zweite Komponente) aufgetragen ist. Dabei gilt, dassje stärker die Datenpunkte über das Koordinatenfeld verteilt sind und je größer der Abstand die Datenpunkte der gealterten Proben von den Datenpunkten der frischen Proben entfernt liegen, um so ausgeprägter ist der Alterungseffekt in Summe und umso aussagekräftiger sind die Ergebnisse des Verfahrens in Summe. Damit dient die PCA insbesondere dem Zweck, festzustellen, ob die ausgewählten Prüfparameter tatsächlich geeignet sind, die angenommene Veränderung der Proben über die Zeit abzubilden. Ist dies nicht der Fall, muss das Set der Prüfparameter entweder verändert oder ergänzt werden.

### Partial Least Square (PLS) Kalibrierungsmethode

Die Partial Least Squares Path Modeling oder Partial Least Squares Structural Equation Modeling (PLS-PM, PLS-SEM) ist eine Methode zur Strukturgleichungsmodellierung, die die Schätzung komplexer Ursache-Wirkungs-Beziehungen in Pfadmodellen mit latenten Variablen ermöglicht.

Es handelt sich dabei um einen komponentenbasierten Schätzungsansatz, der sich von der kovarianzbasierten Strukturgleichungsmodellierung unterscheidet. Im Gegensatz zu diesen Ansätzen der Strukturgleichungsmodellierung passt PLS-PM nicht ein gemeinsames Faktormodell an die Daten an, sondern ein zusammengesetztes Modell und maximiert so die Menge der erklärten Varianz. Darüber hinaus ist PLS-PM durch eine Anpassung in der Lage, auch bestimmte Parameter gemeinsamer Faktormodelle konsistent zu schätzen, und zwar durch einen Ansatz, der als konsistente PLS-PM (PLSc-PM) bezeichnet wird. Eine weitere verwandte Entwicklung ist die faktorbasierte PLS-PM (PLSF), eine Variante, die PLSc-PM als Grundlage für die Schätzung der Faktoren in gemeinsamen Faktormodellen verwendet; diese Methode erhöht die Anzahl der Parameter gemeinsamer Faktormodelle, die geschätzt werden können, beträchtlich und überbrückt effektiv die Lücke zwischen klassischer PLS-PM und kovarianzbasierter Strukturgleichungsmodellierung

Das PLS-PM-Strukturgleichungsmodell setzt sich aus zwei Teilmodellen zusammen: den Messmodellen und dem Strukturmodell. Die Messmodelle stellen die Beziehungen zwischen den beobachteten Daten und den latenten Variablen dar. Das Strukturmodell stellt die Beziehungen zwischen den latenten Variablen dar.

Ein iterativer Algorithmus löst das Strukturgleichungsmodell, indem er die latenten Variablen abwechselnd mit Hilfe des Mess- und des Strukturmodells schätzt, daher auch der Name des Verfahrens: partiell. Das Messmodell schätzt die latenten Variablen als eine gewichtete Summe der manifesten Variablen. Das Strukturmodell schätzt die latenten Variablen mittels einfacher oder multipler linearer Regression zwischen den durch das Messmodell geschätzten latenten Variablen. Dieser Algorithmus wiederholt sich, bis Konvergenz erreicht ist [vgl. auch Avkiram et. al, *"Partial Least Squares Structural Equation Modeling",* Springer Verlag (2018)].

Kurz zusammengefasst liefert die PLS auf der Basis des gleichen Datensatzes ebenfalls ein Koordinatensystem, bei dem für jeden Datenpunkt die Komponente mit der größten Varianz oder Abweichung ("Erste Komponente") gegen die Komponente mit der zweitgrößten Varianz ("Zweite Komponente) aufgetragen ist. Gleichzeitig berechnet sie jedoch für ein Beurteilungskriterium ("Antwort") beispielsweise das Alter der Probe für jeden Datenpunkt einen Wert. Dieser berechnete Wert wird dann in einem Kalibrierungsdiagramm gegen den tatsächlichen Ist-Wert der Probe - also beispielsweise das bekannte Alter - aufgetragen. Durch die Datenwolke wird dann eine Regressionsgerade gelegt, die als Kalibrierungsgerade dient und mit deren Hilfe dann eine unbekannte Probe an Hand ihrer physikochemischen Parameter hinsichtlich ihres Alters eingeordnet werden kann.

### BEISPIELE

### Beispiel 1A/B

### Parameterauswahl und Datengenerierung

Nachfolgend wird das erfindungsgemäße Verfahren für Magermilch (Fettgehalt 0,3 Gew.-%) erläutert. Im ersten Schritt wurden aus den zur Verfügung stehenden Parametern die folgenden ausgewählt:
- gelöste Sauerstoffmenge
- pH-Wert
- Inl = Instabilitätsindex (Lumisizer)
- Helligkeitsfarbwert weiß/schwarz (L*)
- Farbkoordinate rot/grün (a*)
- Farbkoordinate gelb/blau (b*)
- freier Aminostickstoff (PAN)
- Ammoniakgehalt
- Harnstoffgehalt

Insgesamt wurden 4 Magermilch-Proben im Format 0,2 bzw. 1L genommen; diese stellten den ersten Probensatz (P1 bis P4) dar. Die Proben wurden hinsichtlich der ausgewählten Parameter analysiert (Probenalter: 0 Wochen)

Anschließend wurde jede dieser Proben über einen Zeitraum von 22 Wochen bei 20 °C abgedunkelt gelagert. Im Abstand von zwei Wochen wurden aus jeder der ursprünglichen Proben neue Proben (P1.1, P2.1 ... P1) gezogen und ebenfalls gemäß den ausgewählten Parametern analysiert. Auf diese Weise wurden für jede der ursprünglichen 4 Proben 11 Alterungsstufen abgebildet. So bedeutet in der nachfolgenden Tabelle Probe 3 die 3. Probe, frisch abgefüllt, während P3.9 die gleiche Probe in der Alterungsstufe 9 darstellt, also nach einer Lagerung von 18 Wochen. Der so ermittelte Datensatz ist in **Tabelle 1** zusammengefasst.

**Tabelle 1**

| Datensatz für Qualitätsbestimmung | | | | | | | | | | | |
|---|---|---|---|---|---|---|---|---|---|---|---|
| **Probe** | **Format** | **Alter [w]** | **O₂ gelöst [mg/l]** | **pH** | **Inl** | **L*** | **a*** | **b*** | **PAN [mg/l]** | **NH₄ [mg/l]** | **Urea [mg/l]** |
| 1 | 0,2 | 0 | 8 | 6,65 | 0,525 | 89,77 | -5,59 | 10,62 | 25 | 3 | 221 |
| 2 | 1 | 0 | 7,2 | 6,64 | 0,516 | 88,72 | -5,42 | 10,25 | 25 | 2 | 210 |
| 3 | 1 | 0 | 6,74 | 6,63 | 0,522 | 88,05 | -5,01 | 10,13 | 26 | 1 | 206 |
| 4 | 0,2 | 0 | 7,13 | 6,64 | 0,525 | 88,02 | -5,04 | 10 | 25 | 1 | 205 |
| 1.1 | 0,2 | 2 | 5,42 | 6,62 | 0,553 | 88,89 | -5,07 | 10,74 | 28 | 3 | 210 |
| 2.1 | 1 | 2 | 4,67 | 6,62 | 0,563 | 89,17 | -4,83 | 11,06 | 27 | 3 | 220 |
| 3.1 | 1 | 2 | 5,53 | 6,64 | 0,537 | 88,07 | -5,01 | 10,97 | 26 | 2 | 208 |
| 4.1 | 0,2 | 2 | 6,15 | 6,64 | 0,538 | 88,1 | -5,05 | 10,95 | 27 | 2 | 204 |
| 1.2 | 0,2 | 4 | 5,24 | 6,62 | 0,561 | 88,27 | -5,11 | 10,36 | 25 | 3 | 218 |
| 2.2 | 1 | 4 | 4,4 | 6,63 | 0,574 | 88,3 | -4,94 | 10,9 | 26 | 4 | 226 |
| 3.2 | 1 | 4 | 4,65 | 6,62 | 0,547 | 88,58 | -5,33 | 10,63 | 27 | 3 | 231 |
| 4.2 | 0,2 | 4 | 6,13 | 6,62 | 0,547 | 88,6 | -5,36 | 10,39 | 27 | 3 | 237 |
| 1.3 | 0,2 | 6 | 5,5 | 6,6 | 0,563 | 87,88 | -5,14 | 9,61 | 27 | 4 | 222 |
| 2.3 | 1 | 6 | 5,19 | 6,61 | 0,573 | 86,93 | -4,71 | 9,84 | 27 | 5 | 221 |
| 3.3 | 1 | 6 | 5,07 | 6,61 | 0,575 | 88,8 | -5,02 | 11 | 27 | 3 | 209 |
| 4.3 | 0,2 | 6 | 5,99 | 6,62 | 0,545 | 88,79 | -5,1 | 10,92 | 26 | 4 | 206 |
| 1.4 | 0,2 | 8 | 5,16 | 6,6 | 0,566 | 88,32 | -5,05 | 10,26 | 28 | 5 | 232 |
| 2.4 | 1 | 8 | 4,71 | 6,61 | 0,587 | 88,74 | -4,48 | 11 | 25 | 6 | 241 |
| 3.4 | 1 | 8 | 4,73 | 6,61 | 0,567 | 88,05 | -4,99 | 10,77 | 27 | 3 | 211 |
| 4.4 | 0,2 | 8 | 5,7 | 6,62 | 0,581 | 88,19 | -4,92 | 10,83 | 29 | 4 | 214 |
| 1.5 | 0,2 | 10 | 5,29 | 6,6 | 0,573 | 88,35 | -4,98 | 10,15 | 28 | 5 | 224 |
| 2.5 | 1 | 10 | 3,95 | 6,6 | 0,599 | 88,54 | -4,61 | 11,09 | 28 | 6 | 233 |
| 3.5 | 1 | 10 | 5,18 | 6,62 | 0,559 | 88,54 | -4,85 | 10,76 | 26 | 4 | 213 |
| 4.5 | 0,2 | 10 | 5,73 | 6,62 | 0,573 | 88,59 | -4,92 | 10,66 | 16 | 0 | 228 |
| 1.6 | 0,2 | 12 | 4,98 | 6,61 | 0,55 | 87,95 | -5,17 | 10,05 | 27 | 6 | 228 |
| 2.6 | 1 | 12 | 4,08 | 6,61 | 0,588 | 88,35 | -4,7 | 10,89 | 26 | 5 | 205 |
| 3.6 | 1 | 12 | 5,64 | 6,59 | 0,559 | 88,63 | -4,79 | 10,91 | 25 | 1 | 110 |
| 4.6 | 0,2 | 12 | 7,15 | 6,61 | 0,565 | 88,57 | -4,93 | 10,69 | 21 | 4 | 180 |
| 1.7 | 0,2 | 14 | 5,77 | 6,62 | 0,576 | 88,13 | -4,98 | 10,29 | 27 | 6 | 232 |
| 2.7 | 1 | 14 | 4,86 | 6,6 | 0,568 | 88,41 | -4,44 | 10,92 | 27 | 6 | 224 |
| 3.7 | 1 | 14 | 5,67 | 6,58 | 0,571 | 88,45 | -4,72 | 10,75 | 24 | 3 | 183 |
| 4.7 | 0,2 | 14 | 6,31 | 6,6 | 0,573 | 88,34 | -4,83 | 10,54 | 23 | 3 | 232 |
| 1.8 | 0,2 | 16 | 6,04 | 6,62 | 0,578 | 87,75 | -4,64 | 10,5 | 25 | 5 | 230 |
| 2.8 | 1 | 16 | 4,02 | 6,62 | 0,579 | 87,86 | -4,27 | 11,02 | 28 | 6 | 228 |
| 3.8 | 1 | 16 | 5,31 | 6,58 | 0,559 | 88,36 | -4,76 | 10,84 | 24 | 4 | 187 |
| 4.8 | 0,2 | 16 | 6,04 | 6,58 | 0,563 | 88,23 | -4,98 | 10,56 | 27 | 4 | 200 |
| 1.9 | 0,2 | 18 | 5,89 | 6,6 | 0,559 | 88,09 | -4,84 | 10,62 | 30 | 8 | 221 |
| 2.9 | 1 | 18 | 4,65 | 6,59 | 0,585 | 88,24 | -4,47 | 11,05 | 29 | 9 | 230 |
| 3.9 | 1 | 18 | 5,85 | 6,58 | 0,558 | 88,34 | -4,8 | 10,67 | 25 | 4 | 228 |
| 4.9 | 0,2 | 18 | 6,95 | 6,59 | 0,568 | 88,23 | -4,89 | 10,56 | 27 | 5 | 228 |
| 1.10 | 0,2 | 20 | 5,76 | 6,59 | 0,567 | 87,89 | -4,83 | 10,49 | 28 | 8 | 233 |
| 2.10 | 1 | 20 | 4,21 | 6,6 | 0,58 | 88,02 | -4,51 | 10,9 | 27 | 8 | 218 |
| 3.10 | 1 | 20 | 5,48 | 6,58 | 0,564 | 88,16 | -5,22 | 10,54 | 22 | 8 | 211 |
| 4.10 | 0,2 | 20 | 5,32 | 6,59 | 0,563 | 88,33 | -5,04 | 10,96 | 25 | 5 | 177 |
| 1.11 | 0,2 | 22 | 5,87 | 6,59 | 0,571 | 88,14 | -4,8 | 10,76 | 29 | 7 | 218 |
| 2.11 | 1 | 22 | 4,61 | 6,59 | 0,578 | 88,31 | -4,43 | 11,16 | 29 | 9 | 237 |
| 3.11 | 1 | 22 | 5,39 | 6,55 | 0,575 | 88,26 | -4,9 | 10,89 | 27 | 5 | 180 |
| 4.11 | 0,2 | 22 | 5,72 | 6,54 | 0,565 | 88,24 | -4,9 | 10,82 | 27 | 6 | 228 |

### Beispiel 1C

### Überprüfung des Zusammenhangs der Daten mittels PCA = Principal Component Analysis

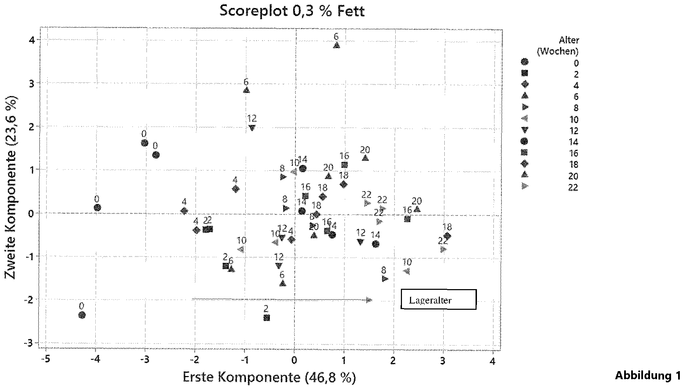

Der dritte Schritt des erfindungsgemäßen Verfahrens ist in **Abbildung 1** wiedergegeben. Dabei wird der zuvor gewonnene Datensatz mit Hilfe der Principal Component Analysis aufbereitet. Die PCA liefert ein Koordinatensystem, welches aus der ersten und zweiten Hauptkomponente gebildet wird. In Abbildung 1 beträgt die erklärte Varianz der Hauptkomponente 1 46.8 %. Die der zweiten Hauptkomponente 23.6 %. Insgesamt erklärt die PCA somit 70.4 % der Varianz in den Originaldaten. Die Kenntnis, welche Parameter die beiden Hauptkomponenten prägen, spielt für das Verfahren keine Rolle, da es in diesem Schritt nur darum geht, die Varianz auf eine skalierbare Größe zu bringen.

Wie man dem Diagramm entnehmen kann, besteht bezogen auf den ursprünglichen Datensatz bei t=0 eine große Verschiebung. Dies bedeutet, dass ein Alterungseffekt tatsächlich vorliegt, der im nachfolgenden vierten Schritt näher untersucht wird. Würden die Datenpunkte alle in unmittelbarer Nachbarschaft der ursprünglichen t=0 Werte liegen, würde dies umgekehrt bedeuten, dass es keinen zeitabhängigen Effekt gibt; das Verfahren würde man daher an dieser Stelle abbrechen. Dieser Schritt dient also dazu, festzustellen, ob das erfindungsgemäße Verfahren auf die zu untersuchenden Produkte überhaupt sinnvoll anzuwenden ist. Falls nicht, müssen die Parameter für die Erhebung des Datensatzes ergänzt oder geändert werden.

### Beispiel 1D

### Aufstellen der PLS = Partial Least Squares-Kalibrierungsmodell

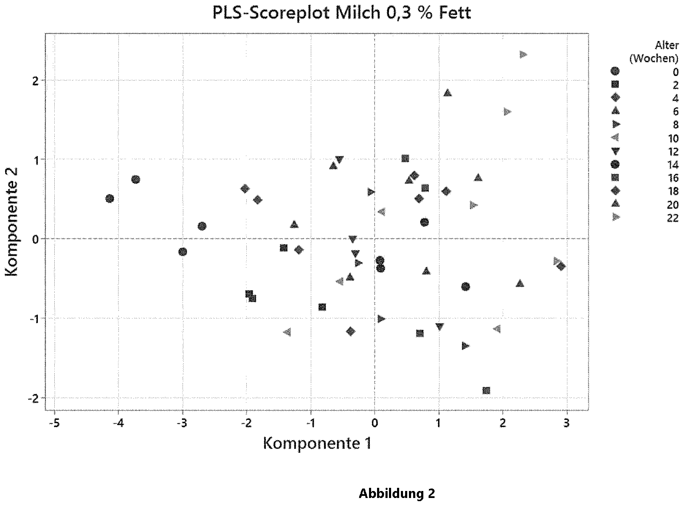

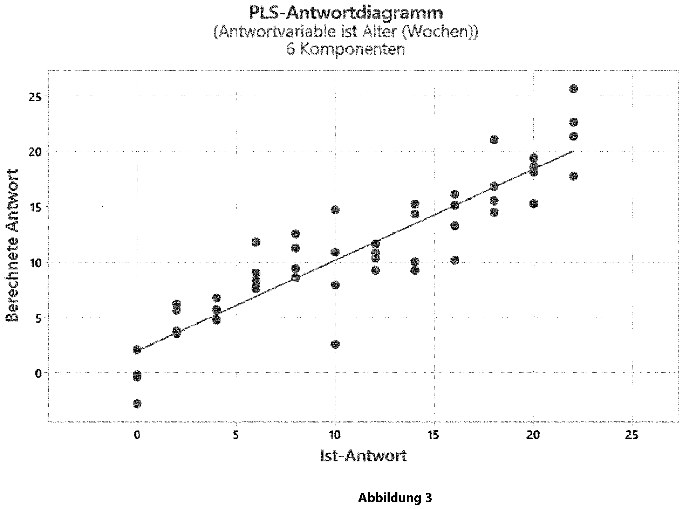

Der vierte Schritt des erfindungsgemäßen Verfahrens ist in den **Abbildungen 2 und 3** wiedergegeben. Nachdem im vorangegangenen Schritt bestätigt wurde, dass das Set an ausgewählten Prüfparametern zu einem Datensatz führt, der tatsächlich eine zeitabhängige Veränderung der Proben widerspiegelt, wird der gleiche Datensatz diesmal mit Hilfe der Partial Least Square Kalibrierungsmethode aufbereitet. Diese liefert wiederum ein Diagramm, bei dem die größte und die zweitgrößte Varianz das Koordinatensystem aufspannen (Abbildung 2), für einen vorgegebenen Parameter ("Antwort") - hier das Alter der Probe - aber auch einen berechneten Messwert liefert. Trägt man die berechneten Messwerte, also das berechnete Alter der Probe, gegen das tatsächliche und ja bekannte Alter dieser Probe auf, kann man durch die Punkteschar eine Kalibrierungskurve legen (Abbildung 3). Mit Hilfe der Kalibrierungskurve kann dann beispielsweise das Alter einer undatierten Probe ermittelt werden.

## Patentansprüche

1. Verfahren zur Beurteilung der Stabilität und Qualität von Milchprodukten, Produkten auf primärer oder hauptsächlicher Basis von Milch inklusive von Käse sowie pflanzlicher Milchalternativen über die Lagerzeit, umfassend oder bestehend aus den folgenden Schritten
(a) Bereitstellen eines ersten Satzes von Proben (P1.0, P2.0 ... Pn.O) eines Milchproduktes, eines Produktes auf primärer oder hauptsächlicher Basis von Milch inklusive von Käse, oder einer pflanzlichen Milchalternative;
(b) Alterung der Proben aus Schritt (a) über definierte Zeiträume unter Erhalt eines n-ten Probensatzes (P1.1, P1.2 ... P2.1, P2.2 ... Pn.x);
(c) Erstellen eines Sets von Prüfparametern;
(d) Anwendung der vorgegebenen Prüfparameter auf jede der einzelnen Proben des ersten und n-ten Probensatzes unter Erzeugung eines Datensatzes;
(e) Überprüfung des Zusammenhangs der Daten im Datensatz mit Hilfe der Principal Component Analysis (PCA) unter Erhalt eines Koordinatensystems (Score-Plot) und Ermittlung der größten Varianz der Daten als erster Komponente und der zweitgrößten Varianz der Daten als zweiter Komponente;
(f) Durchführung einer Partial Least Square Analyse (PLS) mit den Daten.
(g) Generierung eines Partial Least Square (PLS) Kalibrierungsmodells unter Erhalt eines PLS-Antwortdiagramms (Kalibriergerade), wobei die so berechnete Antwort für jeden Datenpunkt gegen die zugehörige gemessene Antwort jedes Datenpunktes in Relation gesetzt wird; sowie
(h) Beurteilung einer fremden Probe bezüglich des festgelegten Beurteilungskriteriums an Hand der Kalibrierungsgraden.

2. Verfahren nach Anspruch 1, **dadurch gekennzeichnet, dass** die Milchprodukte oder pflanzlichen Milchalternativen ausgewählt sind aus der Gruppe, die gebildet wird von Vollmilch, Magermilch, UHT-Milch, Milchpulvern, Sahne, Molke, Quark, Käse, Joghurt oder deren pflanzlichen Alternativen.

3. Verfahren nach den Ansprüchen 1 und/oder 2, **dadurch gekennzeichnet, dass** der erste Probensatz 3 bis 10 Proben umfasst.

4. Verfahren nach mindestens einem der Ansprüche 1 bis 3, **dadurch gekennzeichnet, dass** der zweite Probensatz die Proben des ersten Probensatzes umfasst, von denen jede über einen Zeitraum 1 bis 25 Wochen gelagert worden sind.

5. Verfahren nach mindestens einem der Ansprüche 1 bis 4, **dadurch gekennzeichnet, dass** die Proben ein Volumen zwischen 10 ml und 100 L aufweisen.

6. Verfahren nach mindestens einem der Ansprüche 1 bis 5, **dadurch gekennzeichnet, dass** das Set von Prüfparametern mindestens zwei der folgenden Parameter umfasst:
- gelöste Sauerstoffmenge;
- pH-Wert;
- Instabilitätsindex (Lumisizer);
- Helligkeitsfarbwert weiß/schwarz (L*);
- Farbkoordinate rot/grün (a*);
- Farbkoordinate gelb/blau (b*);
- freier Aminostickstoff (PAN);
- Ammoniakgehalt;
- Harnstoffgehalt;
- Calciumgehalt;
- Säuregehalt;
- rheologisches Verhalten;
- Festigkeit;
- Syneräsegrad; sowie
- thermisches Verhalten (DSC)

7. Verfahren nach Anspruch 6, **dadurch gekennzeichnet, dass** das Set von Prüfparametern mindestens 5, vorzugsweise mindestens 7 Parameter enthält.

8. Verfahren nach mindestens einem der Ansprüche 1 bis 7, **dadurch gekennzeichnet, dass** man als anzuwendendes Beurteilungskriterium das Alter der Probe auswählt.
